(19) Europäisches Patentamt
European Patent Office
Office européen des brevets

(11) EP 1 144 423 B1

(12) **EUROPEAN PATENT SPECIFICATION**

(45) Date of publication and mention of the grant of the patent:
**22.10.2003 Bulletin 2003/43**

(21) Application number: **00900746.9**

(22) Date of filing: **18.01.2000**

(51) Int Cl.[7]: **C07F 15/00**, A61K 31/28

(86) International application number:
**PCT/GB00/00181**

(87) International publication number:
**WO 00/043405 (27.07.2000 Gazette 2000/30)**

(54) **BIS-TERPYRIDINE-PLATINUM(II) COMPLEXES**

BIS-TERPYRIDINPLATINKOMPLEXE

COMPLEXES BIS-TERPYRIDINE-PLATINUM (II)

(84) Designated Contracting States:
**AT BE CH CY DE DK ES FI FR GB GR IE IT LI LU MC NL PT SE**

(30) Priority: **20.01.1999 GB 9901253**

(43) Date of publication of application:
**17.10.2001 Bulletin 2001/42**

(73) Proprietor: **ISIS INNOVATION LIMITED**
**Summertown, Oxford OX2 7BZ (GB)**

(72) Inventor: **LOWE, Gordon**
**Abingdon, Oxfordshire OX14 2HQ (GB)**

(74) Representative:
**Ellis-Jones, Patrick George Armine et al**
**J.A. KEMP & CO.**
**14 South Square**
**Gray's Inn**
**London WC1R 5JJ (GB)**

(56) References cited:
**WO-A-97/27202**

- **LOWE, GORDON ET AL: "Cytotoxicity of 2,2':6',2''-Terpyridineplatinum(II) Complexes against Human Ovarian Carcinoma" J. MED. CHEM. (1999), 42(16), 3167-3174 , 1999, XP002134091**
- **LOWE, GORDON ET AL: "Cytotoxicity of (2,2':6',2''-Terpyridine)platinum(II) Complexes to Leishmania donovani, Trypanosoma cruzi, and Trypanosoma brucei" J. MED. CHEM. (1999), 42(6), 999-1006 , 1999, XP002134092**
- **CHEMICAL ABSTRACTS, vol. 125, no. 12, 16 September 1996 (1996-09-16) Columbus, Ohio, US; abstract no. 157000, LOWE, GORDON ET AL: "A mass spectrometric investigation of the reaction between 4,4'-vinylenedipyridine bis[2,2':6',2''-terpyridine platinum(II)] and the self-complementary oligonucleotide d(CpGpTpApCpG)" XP002134093 & BIOORG. MED. CHEM. (1996), 4(7), 1007-1013 ,1996,**

EP 1 144 423 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Background

This invention relates to a class of bis-2,2':6',2"-terpyridine-platinum (II) complexes (**1**) in which the linker is a bipyridyl of variable length. Such compounds carry a double positive charge on each 2,2':6',2"-terpyridine-platinum (II) moiety and are potent intercalators of DNA. They also platinate guanosine residues at N-7 in double stranded DNA and are effective antitumour agents. The lead compound of this class, 4,4'-vinylenedipyridine bis[2,2':6',2"-terpyridine platinum (II)] (**1**; Z=*trans*-CH=CH-; Y=H) and two 4'-substituted variants of it with 4'-chloro- and 4'-(4-bromophenyl) substituents (**1**; Z=*trans*-CH=CH-; Y=Cl) and (**1**; Z=*trans*-CH=CH-; Y=4-Br.$C_6H_4$) were reported in PCT/GB97/00218 (WO 97/27202) and referred to as $A_{14}$, $I_{14}$ and $Q_{14}$ respectively. The lead compound 4,4'-vinylenedipyridine bis[2,2':6',2"-terpyridine platinum (II)] was more effective than cisplatin for some human ovarian cell lines (e.g. SKOV3) and was more effective against all the cisplatin resistant cell lines tested. This invention results from an investigation of the influence of linker length on the antitumour activity of this class of bis-intercalators.

It has been found that increasing the linker length in general decreases the antitumour activity of the bis-terpyridine Pt(II) complexes and evidence is presented which suggests that the electrostatic stress caused by the proximity of two Pt(II) ions correlates with high antitumour activity. For this reason bis-terpyridine Pt(II) complexes with short linkers are amongst the most effective antitumour agents of this class of compounds.

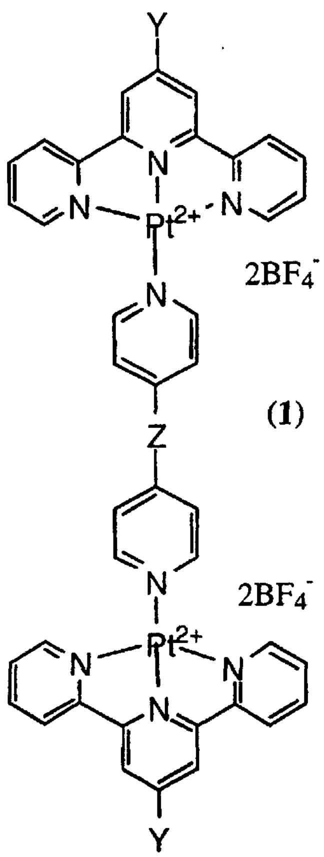

(1)

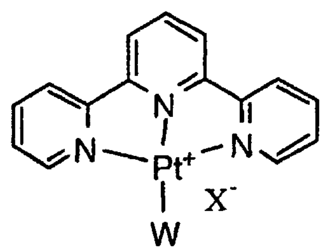

(2)

### Summary of Invention

In one aspect the invention provides a 2, 2':6', 2"-terpyridine Pt(II) complex of the structure

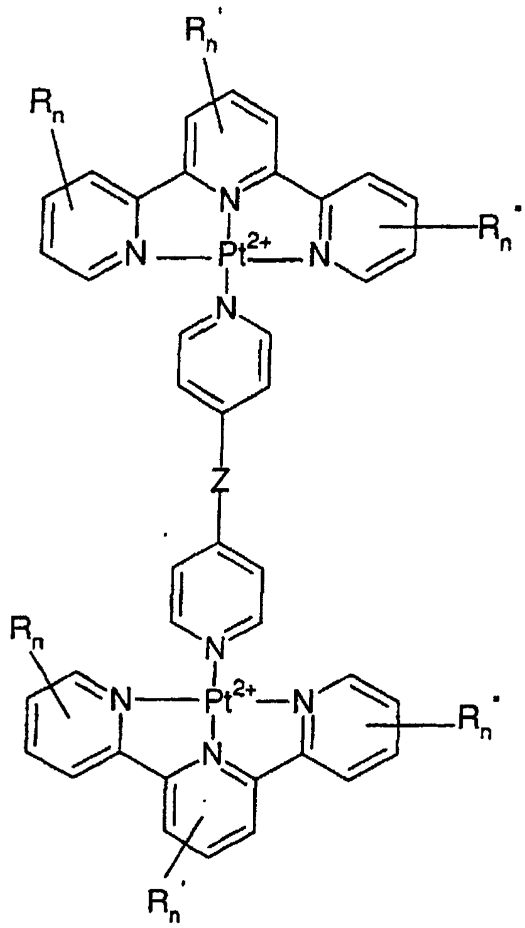

where R, R' and R'' are the same or different and each is H, alkyl, aryl, aralkyl, alkaryl, acyl, halogen, haloalkyl, haloaryl, hydroxyalkyl, hydroxyaryl, aminoalkyl, aminoaryl, primary secondary or tertiary amine, hydrazino, alkylhydrazino, alkoxy, aralkoxy, nitrile, ester, amido, nitro, azido or aziridino,

n is 1, 2 or 3,

Z is a linker composed of 0 to 6 moieties selected from -CH=CH-, -C≡C-, o-, m- or p- $-C_6H_3R'''-$, and Q,

Q is $-C_5H_3R'''N-Pt^{2+}(NH_3)_2-NC_5H_3R'''-$,

R''' is defined as R, R' and R'',

and a counterion to make the complex electrically neutral,

provided that not more than one Q is present,

and provided that Z is not -CH=CH-.

In another aspect the invention provides a method of preparing an anti-tumour agent, which method comprises bringing the complex as defined into a form suitable for administration. In one embodiment R is H, R'' is H, and R' is 4'-H or 4-Cl and n is 1. In another embodiment Z is -C≡C- $(C_6H_4)_m$-C≡C- where m is 0, 1 or 2

## Detailed Description

2,2':6',2''-Terpyridine-platinum(II) complexes were first reported to bind to double stranded DNA by intercalation over twenty years ago, the duplex unwinding angle being comparable to that found with ethidium bromide [1]. An investigation of the binding of hydroxyethanethiolate-2,2':6',2''-terpyridine-platinum(II) (**2**, W=$SCH_2CH_2OH$, $X^-$=$NO_3^-$) with calf-thymus (ct)-DNA by fiber X-ray diffraction techniques showed the platinum ions in the complex to be distributed along the helix axis in accord with the nearest neighbour exclusion model [2]. The fourth ligand to Pt in all the complexes reported were either thiolate or chloride ions so that the complex bore a single positive charge. The binding association constants with ct-DNA were between 0.23 x $10^5$ and 2 x $10^5$ $M^{-1}$, that of the hydroxy-ethanethiolate complex (**2**, W=$SCH_2CH_2OH$, $X^-$=$NO_3^-$) being 0.83 x $10^5$ $M^{-1}$. The chloroplatinum complex **2**(W=Cl, $X^-$=$Cl^-$) had essentially the same binding constant but was shown slowly to form a covalent link to DNA [3]. Recently the hydroxy-platinum complex **2** (W=OH, $X^-$= $BF_4^-$) has been shown to intercalate into DNA with a binding constant of approximately 7 x $10^4$ $M^{-1}$ and

to slowly form a covalent bond with the DNA [4].

4-Picoline-2,2':6',2"-terpyridine-platinum(II) bis-tetrafluoroborate was shown in a ligation assay to unwind and so intercalate into DNA. Circular dichroism was used to determine an equilibrium binding constant of approximately 2x10 $M^{-1}$ for the most stable binding mode of 4-picoline-2,2':6',2"-terpyridine-platinum(II) to poly[d(A-T)$_2$] with a site size of about 4 base pairs, and about 1 x$10^6$ $M^{-1}$ for a second binding mode with a site size of about 2 base pairs. Fluorescence spectroscopy provided further evidence for the strong equilibrium binding constant of 4-picoline-2,2':6', 2"-telpyridine-platinum(II) in that it displaces ethidium bromide bound to DNA. The double positive charge on 4-picoline-2,2':6',2"-terpyridine-platinum(II), together with the intercalative binding mode is probably responsible for the large binding constant [5].

Attempts to obtain crystals suitable for X-ray analysis of the complex of 4-picoline 2,2':6',2"-terpyridine-platinum(II) bis-tetrafluoroborate with the self-complementary oligonucleotide d(CpGpTpApCpG) were unsuccessful. This oligonucleotide was chosen because of the availability of the high resolution structure of its complex with daunomycin, an intercalator of DNA with antitumour activity [6]. Good crystals were obtained, however, from a solution of 4,4'-vinylenedipyridine bis[2,2':6',2"-terpyridine platinum (II)] (**1**; Z=*trans*-CH=CH-; Y=H) and the self-complementary oligonucleotide d(CpGpTpApCpG) but the crystals did not diffract X-rays to high resolution. From a series of NMR experiments it became clear that some nucleosides, especially guanosine, were able to slowly displace 4-picoline from 4-picoline 2,2':6',2"-terpyridine-platinum(II) and the 4,4'-vinylenedipyridine linker from 4,4'-vinylenedipyridine bis[2,2':6',2"-terpyridine platinum (II)] (1; Z=*trans*-CH=CH-; Y=H) [7]. Although it is well established that purine bases, especially guanine, displace chloride ion from Pt(II) derivatives, the best known example being cisplatin {*cis*[$Pt(NH_3)_2Cl_2$]) [8], there does not appear to be any previous report of the displacement of a pyridine-ligand at Pt(II) by a nucleobase. Indeed it has recently been reported that *cis*-[$Pt(NH_3)_2$(pyridine)Cl]$^+$ forms mono functional adducts with DNA by displacing $Cl^-$, the pyridine ligand being perfectly stable [9]. A mass spectrometric investigation, including collisional-induced dissociation tandem mass spectrometry, revealed that the self-complementary oligonucleotide d(CpGpTpApCpG) had been platinated at N-7 of the 3'-terminal guanosine residue. In the light of this entirely unexpected observation the reaction of nucleosides with 4-picoline 2,2':6',2"-terpyridine-platinum(II) was investigated.

All the nucleosides studied (A, G, dA, dG, dC and dT) were found to react with 4-picoline 2,2':6',2"-terpyridine-platinum(ll) to give platinated nucleosides [7]. Guanosine and 2'-deoxyguanosine reacted with 4-picoline 2,2':6',2"-terpyridine-platinum(II) in the most straightforward manner and are platinated at N-7. The adenine nucleosides are bis-platinated at N-1 and N-6 and 2'-deoxycytosine is bis-platinated at N-3 and N-4. The product from deoxythymidine has not been characterised. The bis-platinated nucleosides are totally unexpected, no intermediate monoplatinated species being detected. Although the sites platinated on dA and dC are not available in double stranded DNA because of Watson Crick base-pairing, these compounds may have biologically useful properties if they are incorporated into DNA during replication. What they do show is that N-7 of adenine, which is the only nucleophilic site of this nucleobase exposed in double stranded DNA, is considerably less reactive than the N-1/ N-6 sites which in turn are less reactive than the N-7 site in dG, so providing an explanation of the specificity of platination of dG by 4-picoline 2,2':6',2"-terpyridine-platinum(II) in the self-complementary oligonucleotide d(CpGpTpApCpG).

**Antitumour Activity**

The $IC_{50}$ values of the bis-2,2':6',2"-terpyridine Pt(II) complexes (**1**, Z=*trans*-CH=CH-, Y=H), (**1**, Z=*trans*-CH=CH-, Y=Cl) and (**1**, Z=*trans*-CH=CH-, Y=4-Br.$C_6H_4$), were reported in PCT/GB97/00218 (WO97/27202) and referred to as $A_{14}$, $I_{14}$ and $Q_{14}$ respectively. They were evaluated for *in vitro* cytotoxicity against five human ovarian carcinoma cell lines which included two selected for resistance to cisplatin (Ch1cis$^R$ and A2780cis$^R$) and one for resistance to doxorubicin (Ch1dox$^R$). The compounds were exposed to cells for 96 h and growth inhibition assessed using the sulforhodamine B protein staining assay. These results together with those for the new bis-2,2':6',2"-terpyridine-platinum (II) complexes (Figure 1) are shown in Table 1.

Against the CH1 cell line three of the bis-terpyridine Pt(II) complexes show $IC_{50}$ values below 1μM [$A_{15}$ ($4NO_3^-$), $I_{18}$($4NO_3^-$) and $I_{20}$($6NO_3^-$)] and are comparable in activity to cisplatin. Against the cisplatin resistant CH1 cell line (CH1cis$^R$) four compounds [$A_{14}$ ($4BF_4^-$), $A_{15}$ ($4NO_3^-$), $I_{18}$($4NO_3^-$) and $I_{20}$($6NO_3^-$)] have $IC_{50}$ values below 1μM and are now more effective than cisplatin. It is clear from the resistance factors (RF) that there is little or no cross-resistance with cisplatin. Against the doxirubicin resistant CH1 cell line most of the bis-terpyridine Pt(II) complexes tested have $IC_{50}$ values below 1μM with RF values less than one. The best two compounds are $A_{15}$ ($4NO_3^-$), and $I_{20}$($6NO_3^-$) both with essentially similar $IC_{50}$ values and RF values of 0.6 and 0.8 respectively.

Against the cell line A2780 one compound namely, $I_{15}$ ($4NO_3^-$) has an $IC_{50}$ value below 1μM and is comparable in activity to cisplatin. Against the A2780 cisplatin resistant cell line (A2780cis$^R$) it retains this level of activity (RF 1.1) but is now 13 times more effective than cisplatin.

Against the cell line SKOV3 six of the bis-terpyridine Pt(ll) complexes are more effective than cisplatin, the most effective being [$A_{14}$ ($4BF_4^-$), $I_{15}$ ($4NO_3^-$), and $I_{18}$($4NO_3^-$)] each with comparable antitumour activity.

This investigation was undertaken to explore the effect of linker length on the antitumour activity of bis-terpyridine Pt(II) complexes. Since $I_{16}(4NO_3^-)$ and $I_{17}(4NO_3^-)$ are not amongst the most effective agents against any of the cell lines, longer chain lengths do not apparently improve antitumour activity. By contrast, compounds with the shortest linker length are amongst the most effective against three cell lines, e.g. $I_{18}(4NO_3^-)$ against CH1, CH1cis$^R$, CH1dox$^R$ and SKOV3. The exception to this conclusion is $A_{20}(6NO_3^-)$ and $I_{20}(6NO_3^-)$ which have long linkers and yet are very effective against most cell lines. This implies that activity may well correlate with the electrostatic stress within these molecules caused by the double positive charge on each of the Pt(II). In general the shorter the chain length the higher the electrostatic stress will be within the molecule. Since this is expected to increase the rate of platination of DNA and/or other targets for these antitumor agents, and thereby release the stress, this could account for the effectiveness of the bis-terpyridine Pt(II) complexes in general. Although $A_{20}(6NO_3^-)$ and $I_{20}(6NO_3^-)$ have long linkers, the electrostatic stress is high in these molecules since they possess a third Pt(II) in the middle of the chain and thus carry six positive charges. Indeed the electrostatic stress in these molecules is expected to be similar to that in $A_{18}(4NO_3^-)$and $I_{18}(4NO_3^-)$ where the distance between Pt(II)s is comparable.

The counterions used for these bis-2,2':6',2"-terpyridine Pt(II) complexes is profoundly significant. First, they have a dramatic influence on the solubility of the complexes in different solvents. For good solubility of the complexes in water, nitrate ions are a suitable choice. All of the new complexes reported in Table 1 have nitrate ions as the counterions and they are all sufficiently water soluble for administration in this solvent alone. Secondly, because the solubility of the bis-2,2':6',2"-terpyridine Pt(II) complexes in solvents of different polarity is influenced by the nature of the counterions and since penetration of cells depends on the partitioning of the complexes between hydrophilic and hydrophobic environments, the nature of the counterions can have a significant influence on the antitumour activity. In Table 1 a comparison can be made between $A_{14}$ with $4BF_4^-$ counterions and $4NO_3^-$ counterions. What is seen is that with $4BF_4^-$ counterions the complex is more effective than the $4NO_3^-$ complex against CH1, CH1cis$^R$, A2780 and A2780cis$^R$ and SKOV3 cell lines but against CH1dox$^R$ the $4NO_3^-$ complex is preferred. Thus the counterion giving optimal antitumour activity against a particular tumour will depend in part in its ability to modulate the partitioning between hydrophobic and hydrophilic media of the chosen bis-2,2':6',2"-terpyridine Pt(II) complex. Other counterions sufficiently non-nucleophilic to be compatible with the Pt(II) complexes include sulphate, carbonate, phosphate, pyrophosphate or other phosphorus oxyanion or ester thereof, sulphonates and carboxylates. Preferred counterions are non-toxic.

The groups R, R', R" and R"' may also be useful in modulating the partitioning of a complex between hydrophobic and hydrophilic media.

2,2':6',2"-Terpyridine Pt(II) complexes and especially bis-2,2':6',2"-terpyridine Pt(II) complexes form stable conjugates with anionic polymers and dendrimers. Such conjugates form another aspect of the present invention. Thus $I_{18}$ when loaded alone onto a Sephadex G25 column is retained by the column on elution, whereas when it is mixed with the anionic Starburst (PANAM) dendrimer Generation 3.5 before loading it is eluted from the column in the void volume, thus demonstrating tight ion pair conjugation. There is now unequivocal evidence that many different macromolecules passively diffuse into and accumulate within solid tumours. The phenomenon has been studied extensively by Maeda et al. who termed tumour accumulation of macromolecules as the 'enhanced permeability and retention effect' (EPR) attributing it to two factors [11]. First the tumour vasculature has a discontinous endothelium (it is therefore leaky) and allows macromolecules to enter the tumour cell, whereas normal cells with a continuous endothelial barrier exclude macromolecules. Secondly, there is a lack of effective lymphatic drainage in tumours preventing clearance of the macromolecules that have entered the cells and so they accumulate within the tumour. Thus the EPR effect provides a mechanism for the highly selective delivery of drug macromolecular conjugates. This can be achieved with the 2,2': 6',2"-terpyridine Pt(II) and bis-2,2':6',2"-terpyridine Pt(II) complexes by forming a conjugate with an anionic macromolecule. Anionic polymers and dendrimers carrying many negatively charged ions can thus deliver selectively to tumours cells many 2,2':6',2"-terpyridine Pt(II) or bis-2,2':6',2"-terpyridine Pt(II) complexes per macromolecule and thus greatly improve the therapeutic index.

An important mechanism of resistance to antitumour agents is the down regulation of the apoptotic system. It has been shown [12], that the Bcl-2 gene is a major player in this process since by over-expression of this gene the tumour cell becomes refractive to antitumour agents. This refractivity can be reversed, however, by the use of antisense agents which bind to the mRNA and down regulate the expression of the Bcl-2 gene. The down regulation of the Bcl-2 gene combined with the antitumour activity of the bis-2,2':6',2"-terpyridine Pt(II) complexes thus provides a new approach of overcoming potential resistance to this new class of antitumour agents.

Thus the present invention also provides a preparation suitable for use in the treatment of cancer which comprises a complex or conjugate of the present invention, optionally with an agent which down-regulates expression of Bcl-2 gene.

In a recent clinical trial a combination therapy of three drugs (cyclophosphamide, doxorubicin and cisplatin) was compared with the optimal dose of a single agent (carboplatin) for patients with advanced ovarian cancer. No difference was found in survival between groups on the combination therapy regimen and those on carboplatin. Carboplatin, unlike the combination therapy, however, has few side effects. The investigators concluded that carboplatin is a safe, effective and appropriate standard treatment for women with advanced ovarian cancer [13].

The last row in Table 1 shows the $IC_{50}$ values of carboplatin, currently the most widely used drug for ovarian cancer. Most of the bis-2,2':6',2"-terpyridine Pt(II) complexes reported in Table 1 are significantly more effective inhibitors (i.e. lower $IC_{50}$ values) than carboplatin against human ovarian cell line growth *in vitro.* The maximum tolerated dose was determined for $A_{15}(4NO_3)$ and $I_{15}(4NO_3)$.

Drugs were administered as a single intraperitoneal injection at 25, 50 and 100 mg/kg. Animals were observed daily for signs of induced toxicity and body weight determined 2 weeks post-dosing. There were no deaths at any of the three doses and no loss of body weight. $A_{15}(4NO_3)$. gave no sign of toxicity or body weight loss even at 100 mg/kg. $I_{15}(4NO_3)$ at 100 mg/kg gave some indication of toxicity (mild ataxia/twitching) but not at 50 mg/kg.

## EXPERIMENTAL

By way of example the synthesis and characterisation of the several bis[2,2':6',2"-terpyridine platinum (II)] complexes are provided. The structures of these complexes are shown in Figure 1.

### General procedures

Solvents (A.R. and h.p.l.c. grade) were purchased from Aldrich Chemical Company and Rathburn Chemicals. Diethylamine was dried over potassium hydroxide pellets, distilled from potassium hydroxide and stored under argon over potassium hydroxide pellets. Water refers to deionised water.

Melting points were determined on a Reichert heating stage and are uncorrected.

H n.m.r. spectra were recorded on a Varian Gemini 200MHz spectrometer or a Bruker AM 500 MHz spectrometer at 300K. Samples run in deuterochloroform ($CDCl_3$) were referenced to the solvent (7.26 ppm). Samples run in deuterium oxide ($D_2O$) were referenced to dioxane (3.75 ppm). Samples run in deuterated dimethylsulfoxide (DMSO) were referenced to the solvent (2.50 ppm). Chemical shifts are expressed in ppm. Abbreviations for multiplicity are: s, singlet; d, doublet; t, triplet; q, quartet; m, multiplet; br s, broad singlet; br d, broad doublet. Relative intensities are expressed as the number of protons such that "2H" denotes a relative intensity of two protons. $^1H$ n.m.r. spectra are expressed in order of chemical shift, multiplicity, coupling constant, relative intensity and assignment.

Flash chromatography was performed by using h.p.l.c. grade solvents and Merck silica gel 60 (230-400 mesh ASTM). Thin layer chromatography was performed on Merck precoated aluminium t.l.c. plates coated with silica gel 60F 254 (0.2 mm) and visualised by means of ultraviolet light.

Electrospray mass spectroscopy was carried out on a VG Biotech Bio-Q spectrometer using a dilute solution of the sample in methanol/water.

2-Methyl-4-(4-pyridyl)-3-butyn-2-ol was synthesised by the method of Della Ciana and Haim [14].

### Preparation of 1,4-bis(4-pyridyl)butadiyne 4-Ethynylpyridine.

A solution of 2-methyl-4-(4-pyridyl)-3-butyn-2-ol (4.00 g, 24.8 mmol) and sodium hydroxide (0.80 g, 20.0 mmol) in toluene (120 ml) was heated at reflux for 4 h and then allowed to cool to room temperature. The mixture was decolourised by shaking with activated charcoal and filtered. The clear yellow filtrate was carefully evaporated to dryness under reduced pressure (30 mmHg, water bath temperature 30°C) to yield 4-ethynylpyridine (2.23 g, 87%) as a crystalline white solid which was used immediately in the next step without further purification. The purity of the solid was checked by analysis of the $^1H$ n.m.r. spectrum. $^1H$ n.m.r. {200 MHz, $CDCl_3$}: δ 8.61, AA'm, 2H, H2, H6; 7.37, BB'm, 2H, H3, H5; 3.31, s, 1H, acetylenic H.

### 1,4-Bis(4-pyridyl)butadiyne.

Copper(I) chloride (131 mg, 1.32 mmol) was added to a solution of 4-ethynylpyridine (1.40 g, 13.6 mmol) in pyridine (50 ml) and shaken in an atmosphere of oxygen for 4 h in darkness. The mixture was poured into cold water (300 ml) where upon a white solid precipitated. The solid was filtered, washed with water and taken up in chloroform. The solution was dried ($MgSO_4$ and the solvent removed under reduced pressure to yield a white solid (1.07 g). Recrystallisation from 40:60 petroleum ether/dichloromethane afforded 1,4-bis(4-pyridyl)butadiyne (774 mg, 28%) as white crystalline plates, m.p. 208-209°C (lit.[1] 203-205 °C). $^1H$ n.m.r. {200 MHz, $CDCl_3$}: δ 8.63, AA'm, 4H, H2, H6; 7.38, BB'm, 4H, H3, H5.

**Preparation of 4,4"-dipyridyl-1',4'-diethynylbenzene 1,4-Diethynylbenzene**

1,4-Bis(trimethylsilylethynyl)benzene (1.00 g, 3.71 mmol) was dissolved in dichloromethane (15 ml) and methanol (15 ml). Aqueous potassium hydroxide (1 M, 3 ml) was added dropwise with stirring. The mixture was stirred at room temperature for 2.5 h. The organic solvent was removed under reduced pressure. Water (25 ml) was added and the mixture was extracted with ether (3x30 ml). The combined ether extracts were dried ($MgSO_4$), decolourised with activated charcoal and the solvent was removed under reduced pressure to yield 1,4-diethynylbenzene (367 mg, 78%) as a white solid which was used immediately without further purification, m.p. 95-96 °C. $^1H$ n.m.r. (200 MHz, $CDCl_3$): δ 7.46, s, 4H, $C_6H_4$; 3.18, s, 2H, acetylenic H.

**4,4"-Dipyridyl-1',4'-diethynylbenzene**

Bis(triphenylphosphine)palladium(II) chloride (70.2 mg, 0.10 mmol) was added to a stirred suspension of 4-bromopyridine hydrochloride (778 mg, 4.0 mmol), 1,4-diethynylbenzene (252 mg, 2.0 mmol), copper(I) iodide (28.6 mg, 0.15 mmol) and dry diethylamine (15 ml) under an atmosphere of argon. The reaction was stirred for 36 h at room temperature under argon. A further portion of bis(triphenylphosphine)palladium(II) chloride (16.3 mg, 0.023 mmol) was added. The reaction was stirred for a further 48 h. The mixture was filtered through celite using ethyl acetate and concentrated under reduced pressure. The residue was chromatographed on flash silica gel using 5% methanol/dichloromethane as eluant and further purified by chromatography on alumina (grade IV) using 50% ethyl acetate/petroleum ether (40-60°C) as eluant to yield 4,4"-dipyridyl-1',4'-diethynylbenzene (284 mg, 51%) as a pale yellow solid, m.p. 185-203°C. $^1H$ n.m.r. (200 MHz, $CDCl_3$): δ 8.64, AA'm, 4H, H2, H6, H2", H6"; 7.58, s, 4H, $C_6H_4$; 7.41, BB'm, 4H, H3, H5, H3", H5".

**Synthesis of 4",4'"-dipyridyl-4,4'-diethynylbiphenyl 4,4'-Diethynylbiphenyl**

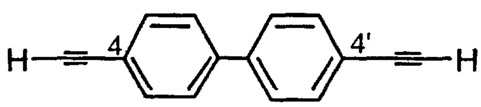

4,4'-Bis(trimethylsilylethynyl)biphenyl (1.04 g, 2.99 mmol) was dissolved in dichloromethane (15 ml) and methanol (15 ml). Aqueous potassium hydroxide (1M, 3 ml) was added dropwise with stirring. The mixture was stirred at room temperature for 2.5 h. The organic solvent was removed under reduced pressure. Water (25 ml) was added and the mixture was extracted with ether (3x30 ml). The combined ether extracts were dried ($MgSO_4$), decolourised with activated charcoal and the solvent was removed under reduced pressure to yield 4,4'-diethynylbiphenyl (562 mg, 93%) as a white solid which was used immediately without further purification, m.p. 167-169 °C. $^1H$ n.m.r. (200 MHz, $CDCl_3$) δ 7.57, s, 8H, $2xC_6H_4$; 3.15, s, 2H, acetylenic H.

**4,4'-Diethynyl-4",4'"-dipyridylbiphenyl**

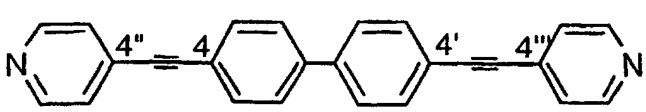

Bis(triphenylphosphine)palladium(II) chloride (78.2 mg, 0.11 mmol) was added to a stirred suspension of 4-bromopyridine hydrochloride (846 mg, 4.35 mmol), 4,4'-diethynylbiphenyl (440 mg, 2.18 mmol), copper(I) iodide (29.2 mg, 0.15 mmol) and dry diethylamine (30 ml) under an atmosphere of argon. The reaction was stirred for 48 h at room temperature under argon. The mixture was filtered through celite using ethyl acetate and concentrated under reduced pressure. The residue was chromatographed on flash silica gel using 5% methanol/dichloromethane as eluant and further purified by chromatography on alumina (grade IV) using 50% ethyl acetate/petroleum ether (40-60 °C) as

eluant to yield 4,4'-diethynyl-4",4'"-dipyridylbiphenyl (310 mg, 20%) as a pale yellow solid, m.p. 205-275 °C. $^1$H n.m.r. (200 MHz, $CDCl_3$): δ 8.63, AA'm, 4H, H2", H6", H2'", H6'"; 7.65, s, 8H, $2xC_6H_4$; 7.41, BB'm, 4H, H3", H5", H3'", H5'".

**4,4'-Dipyridine bis[(2,2':6',2"-terpyridine)platinum(II)] tetranitrate ($A_{18}$).**

A solution of silver nitrate (64.6 mg, 0.38 mmol) in acetone/water (4:1, 0. 5 ml) was added dropwise to a suspension of diiodo-1,5-cyclooctadieneplatinum(II) (99.6 mg, 0.18 mmol) in acetone/water (4:1, 0.75 ml). The mixture was vortexed and sonicated for a few minutes and then centrifuged. The silver iodide precipitate was discarded. The supernatant was added to a suspension of 2,2':6',2"-terpyridine (33.6 mg, 0.144 mmol) in acetonitrile (0.3 ml). The mixture was vortexed and sonicated for a few minutes and then centrifuged. The supernatant was removed and discarded. The pellet was washed with acetonitrile/ether (1:3, 3x1.5 ml) and then dissolved in water (1.5 ml). A solution of 4,4'-dipyridine (11.3 mg, 0.072 mmol) in acetone (0.75 ml) was added. The mixture was vortexed and sonicated for 1 h. The mixture was added to ether/acetone (3:7, 25 ml) to precipitate the complex. The mixture was centrifuged and the pellet washed with ether/acetone (3:1, 3x20 ml) to yield 4,4'-dipyridine bis[(2,2':6',2"-terpyridine)platinum(II)] tetranitrate (86 mg, 95%) as a yellow solid. The product was purified by re-precipitation from ether/acetone (1:1, 20 ml). $^1$H n.m.r. {500 MHz, $D_2O$}: δ 9.43, AA'm, 4H, H2'", H6'"; 8.55, t, *J* 8.2 Hz, 2H, H4'; 8.40-8.47, series of multiplets, 16H, H3'", H5'", H3', H5', H3, H3", H4, H4"; 7.90, d, *J* 5.6 Hz, 4H, H6, H6"; 7.74, m, 4H, H5, H5". ESMS (CV=5 V): *m/z* 253.2 ($M^{4+}$, 100%), 292.2 {$[M\text{-}Pt(terpy)]^{2+}$, 52%}.

***trans*-4,4'-Vinylidenedipyridine bis[(2,2':6',2"-terpyridine)platinum(II)] tetranitrate ($A_{14}$, X= $NO_3^-$).** [NOT CLAIMED]

A solution of silver nitrate (64.6 mg, 0.38 mmol) in acetone/water (4:1, 0. 5 ml) was added dropwise to a suspension of diiodo-1,5-cyclooctadieneplatinum(II) (99.6 mg, 0.18 mmol) in acetone/water (4:1, 0.75 ml). The mixture was vortexed and sonicated for a few minutes and then centrifuged. The silver iodide precipitate was discarded. The supernatant was added to a suspension of 2,2':6',2"-terpyridine (33.6 mg, 0.144 mmol) in acetonitrile (0.3 ml). The mixture was vortexed and sonicated for a few minutes and then centrifuged. The supernatant was removed and discarded. The pellet was washed with acetonitrile/ether (1:3, 3x1.5 ml) and then dissolved in water (1.5 ml). A solution of *trans*-4,4'-vinylidenedipyridine (13.1 mg, 0.072 mmol) in acetone (0.75 ml) was added. The mixture was vortexed and sonicated for 1 h. The mixture was added to ether/acetone (3:7, 25 ml) to precipitate the complex. The mixture was centrifuged and the pellet washed with ether/acetone (3:1, 3x20 ml) to yield trans-4,4'-vinylidenedipyridine bis[(2,2': 6',2"-ter-pyridine)platinum(II)] tetranitrate (90.6 mg, 98%) as an apricot-coloured solid. The product was purified by re-precipitation from ether/acetone (1:1, 20 ml). $^1$H n.m.r. {500 MHz, $D_2O$}: δ 9.18, AA'm, 4H, H2'", H6'"; 8.53, t, *J* 8.2 Hz, 2H, H4'; 8.16, BB'm, 4H, H3'", H5'"; 7.89-7.91, s and d, 6H, vinylic-H, H6, H6"; 8.38-8.45, series of multiplets, 12H, H3', H5', H3, H3", H4, H4"; 7.74, m, 4H, H5, H5". ESMS (CV=5 V): *m/z* 259.7 ($M^{4+}$, 100%), 305.2 {$[M\text{-}Pt(terpy)]^{2+}$, 15%}.

**1,4-Bis(4-pyridyl)butadiyne bis[(2,2':6',2"-terpyridine)platinum(II)] tetranitrate ($A_{15}$).**

A solution of silver nitrate (64.6 mg, 0.38 mmol) in acetone/water (4:1, 0. 5 ml) was added dropwise to a suspension of diiodo-1,5-cyclooctadieneplatinum(II) (99.6 mg, 0.18 mmol) in acetone/water (4:1, 0.75 ml). The mixture was vortexed and sonicated for a few minutes and then centrifuged. The silver iodide precipitate was discarded. The supernatant was added to a suspension of 2,2':6',2"-terpyridine (33.6 mg, 0.144 mmol) in acetonitrile (0.3 ml). The mixture was vortexed and sonicated for a few minutes and then centrifuged. The supernatant was removed and discarded. The pellet was washed with acetonitrile/ether (1:3, 3x1.5 ml) and then dissolved in water (1.5 ml). A solution of 1,4-bis(4-pyridyl)butadiyne (14.7 mg, 0.072 mmol) in acetone (0.75 ml) was added. The mixture was vortexed and sonicated for 1 h. The mixture was added to ether/acetone (3:7, 25 ml) to precipitate the complex. The mixture was centrifuged and the pellet washed with ether/acetone (3:1, 3x20 ml) to yield 1,4-bis(4-pyridyl)butadiyne bis[(2,2':6',2"-terpyridine)platinum(II)] tetranitrate (92.7 mg, 98%) as a light brown solid. The product was purified by re-precipitation from ether/acetone (1:1, 20 ml). $^1$H n.m.r. {500 MHz, $D_2O$}: δ 9.23, AA'm, 4H, H2'", H6'"; 8.53, t, *J* 8.2 Hz, 2H, H4'; 8.40-8.45, series of multiplets, 12H, H3', H5', H3, H3", H4, H4"; 8.08, BB'm, 4H, H3'", H5'"; 7.86, d, *J* 5.6 Hz, 4H, H6, H6"; 7.73, m, 4H, H5, H5". ESMS (CV=5 V): *m/z* 265.2 ($M_{4+}$, 100%), 316.2 {$[M\text{-}Pt(terpy)]^{2+}$, 58%}.

**Synthesis of 4'-chloro-2,2':6'2"-terpyridine platinum(II) complexes 4,4'-Dipyridine bis[(4'-chloro-2,2':6',2"-terpyridine)platinum(II)] tetranitrate ($I_{18}$)**

A solution of silver nitrate (64.6 mg, 0.38 mmol) in acetone/water (4:1, 0. 5 ml) was added dropwise to a suspension of diiodo-1,5-cyclooctadieneplatinum(II) (99.6 mg, 0.18 mmol) in acetone/water (4:1, 0.75 ml). The mixture was vortexed and sonicated for a few minutes and then centrifuged. The silver iodide precipitate was discarded. The

supernatant was added to a suspension of 4'-chloro-2,2':6',2"-terpyridine (38.6 mg, 0.144 mmol) in acetonitrile (0.3 ml). The mixture was vortexed and sonicated for a few minutes and then centrifuged. The supernatant was removed and discarded. The pellet was washed with acetonitrile/ether (1:3, 3x1.5 ml) and then dissolved in water (1.5 ml). A solution of 4,4'-dipyridine (11.3 mg, 0.072 mmol) in acetone (0.75 ml) was added. The mixture was vortexed and sonicated for 1 h. The mixture was added to ether/acetone (3:7, 25 ml) to precipitate the complex. The mixture was centrifuged and the pellet washed with ether/acetone (3:1, 3x20 ml) to yield 4,4'-dipyridine bis[(2,2':6',2"-terpyridine)platinum(II)] tetranitrate (95.0 mg, 99%) as a yellow solid. The product was purified by re-precipitation from ether/acetone (1:1, 20 ml). $^1$H n.m.r. {500 MHz, $D_2O$}: δ 9.42, AA'm, 4H, H2''', H6'''; 8.60, s, 4H, H3', H5'; 8.43-8.48, series of multiplets, 12H, H3''', H5''', H3, H3", H4, H4"; 7.93, d, *J* 5.6 Hz, 4H, H6, H6"; 7.78, m, 4H, H5, H5". ESMS (CV=10 V): *m*/*z* 270.4 ($M^{4+}$, 100%).

**1,4-Bis(4-pyridyl)butadiyne bis[(4'-chloro-2,2':6'2"-terpyridine)platinum(II)] tetranitrate ($I_{15}$)**

A solution of silver nitrate (37.0 mg, 0.218 mmol) in acetone/water (4:1, 0.5 ml) was added dropwise to a suspension of diiodo-1,5-cyclooctadieneplatinum(II) (56.6 mg, 0.102 mmol) in acetone/water (4:1, 0.5 ml). The mixture was vortexed and sonicated for a few minutes and then centrifuged. The silver iodide precipitate was discarded. The supernatant was added to a suspension of 4'-chloro-2,2':6',2"-terpyridine (21.9 mg, 0.082 mmol) in acetonitrile (0.25 ml). The mixture was vortexed and sonicated for a few minutes and then centrifuged. The supernatant was removed and discarded. The pellet was washed with acetonitrile/ether (1:3, 2x1.5 ml) and then dissolved in water (0.75 ml). A solution of the 1,4-bis(4-pyridyl)butadiyne (8.7 mg, 0.043 mmol) in methanol (0.75 ml) was added. The mixture was vortexed and sonicated for 15 min and allowed to stand at room temperature for 30 minutes. The solution was added dropwise to ether/acetone (1:1, 20 ml) to precipitate the complex. The mixture was centrifuged. The pellet was washed with ether/acetone (1:1, 4x20 ml) and then dried to yield the desired complex (58.4 mg, 99%) as a dusky orange solid. $^1$H n.m.r. {500 MHz, $D_2O$}: δ 9.22, AA' m, 4H, H2''', H6'''; 8.58, s, 4H, H3', H5'; 8.48-8.34, series of multiplets, 8H, H3, H3", H4, H4"; 8.08, $BB^1$ m, 4H, H3''', H5'''; 7.89, d, J5.7 Hz, 4H, H6, H6"; 7.76, m, 4H, H5, H5". ESMS (CV=10 V): *m*/*z* 282.7 ($M^{4+}$, 100%), 333.8 {$[M\text{-}Pt(Cl\text{-}terpy)]^{2+}$, 80%}.

**4,4"-Dipyridyl-1',4'-diethynylbenzene-bis[(4'-chloro-2,2';6'2"-terpyridine)platinum(II)] tetranitrate ($I_{16}$)**

A solution of silver nitrate (35.3 mg, 0.208 mmol) in acetone/water (4:1, 0.5 ml) was added dropwise to a suspension of diiodo-1,5-cyclooctadieneplatinum(II) (56.0 mg, 0.101 mmol) in acetone/water (4:1, 0.5 ml). The mixture was vortexed and sonicated for a few minutes and then centrifuged. The silver iodide precipitate was discarded. The supernatant was added to a suspension of 4'-chloro-2,2':6',2"-terpyridine (21.4 mg, 0.080 mmol) in acetonitrile (0.25 ml). The mixture was vortexed and sonicated for a few minutes and then centrifuged. The supernatant was removed and discarded. The pellet was washed with acetonitrile/ether (1:3, 2x1.5 ml) and then dissolved in water (0.75 ml). A solution of the 4,4"-dipyridyl-1',4'-diethynylbenzene (11.3 mg, 0.043 mmol) in methanol (0.75 ml) was added. The mixture was vortexed and sonicated for 15 min and allowed to stand at room temperature for 30 minutes. A yellow precipitate formed. The mixture was centrifuged and the pellet washed with ether/acetone (1:1, 3x20 ml) to yield the desired complex (33.4 mg) as a yellow solid. The supernatant was added dropwise to ether/acetone (1:1, 20 ml) to precipitate more of the complex which was washed with ether/acetone (1:1, 4x20 ml) and then dried to yield the desired complex (22.2 mg) as a yellow solid. The total yield of the product was 55.6 mg (96%). $^1$H n.m.r. {500 MHz, $D_2O$}: δ 9.16, AA' m, 4H, H2''', H6'''; 8.58, s, 4H, H3', H5'; 8.48-8.40, series of multiplets, 8H, H3, H3", H4, H4"; 8.02, BB' m, 4H, H3''', H5'''; 7.93, d, *J* 5.7 Hz, 4H, H6, H6"; 7.80, s, 4H, H2'''', H3'''', H5'''', H6''''; 7.77, m, 4H, H5, H5". ESMS (CV=10 V): *m*/*z* 301.7 ($M^{4+}$, 100%), 371.8 {$[M\text{-}Pt(Cl\text{-}terpy)]^{2+}$, 38%}.

**4,4'-Diethynyl-4",4'''-dipyridylbiphenyl-bis[(4'-chloro-2,2':6'2"-terpyridine)platinum(II)] tetranitrate ($I_{17}$)**

A solution of silver nitrate (37.1 mg, 0.218mmol) in acetone/water (4:1, 0.5 ml) was added dropwise to a suspension of diiodo-1,5-cyclooctadieneplatinum(II) (56.5 mg, 0.102 mmol) in acetone/water (4:1, 0.5 ml). The mixture was vortexed and sonicated for a few minutes and then centrifuged. The silver iodide precipitate was discarded. The supernatant was added to a suspension of 4'-chloro-2,2':6',2"-terpyridine (21.4 mg, 0.080 mmol) in acetonitrile (0.25 ml). The mixture was vortexed and sonicated for a few minutes and then centrifuged. The supernatant was removed and discarded. The pellet was washed with acetonitrile/ether (1:3, 2x1.5 ml) and then dissolved in water (0.75 ml). A solution of the 4,4'-diethynyl-4",4'''-dipyridylbiphenyl (14.3 mg, 0.040 mmol) in methanol (1.5 ml) was added. The mixture was vortexed and sonicated for 15 min and allowed to stand at room temperature for 30 minutes. The dark solution was added dropwise to ether/acetone (1:1, 20 ml) to precipitate the complex which was washed with ether/acetone (1:1, 4x20 ml) and then dried to yield the desired complex (37.6 mg, 61%) as a yellow solid. $^1$H n.m.r. {500 MHz, $D_2O$}: δ 9.10, AA' m, 4H, H2''', H6'''; 8.56, s, 4H, H3', H5'; 8.45-8.38, series of multiplets, 8H, H3, H3", H4, H4"; 8.02, BB' m,

4H, H3''', H5'''; 7.92, d, *J* 5.7 Hz, 4H, H6, H6''; 7.80, m, 4H, H5, H5''; 7.79, s, 8H, H2'''', H3'''', H5'''', H6''''. ESMS (CV=10 V): *m*/*z* 320.8 ($M^{4+}$, 100%), 410.0 {[M-Pt(Cl-terpy)]$^{2+}$, 69%}.

**Extended linkers based on *trans*-platin. *trans*-Diamminebis(4,4'-dipyridyl)platinum(II) dinitrate.**

A solution of silver nitrate (36.3 mg, 0.200 mmol) in acetone/water (1:1, 0.5 ml) was added to a suspension of *trans*-platin (30.0 mg, 0.100 mmol) in acetone/water (1:1, 0.5 ml) and sonicated for 24 h. The mixture was centrifuged and the supernatant was added to a solution of 4,4'-dipyridyl (31.2 mg, 0.200 mmol) in acetone (0.5 ml), maintaining an excess of 4,4'-dipyridyl. The mixture was sonicated for 24 h and then centrifuged to yield trans-diamminebis(4,4'-dipyridyl)platinum(II) dinitrate(64.9 mg, 97%) as a white solid. $^1$H n.m.r. {500 MHz, DMSO}: δ 8.90, AA'm, 4H, H2, H6; 8.84, AA'm, 4H, H2', H6'; 8.25, BB'm, 4H, H3, H5; 7.98, BB'm, 4H, H3', H5'; 4.73, b s, 6H, $NH_3$. ESMS (CV=30 V): *m*/*z* 270.8 ($M^{2+}$, 60%), 540.3 [$(M-H)^+$, 33%].

***trans*-Diamminebis[1,4-bis(4-pyridyl)butadiyne]platinum(II) dinitrate.**

A solution of silver nitrate (28.0 mg, 0.165 mmol) in acetone/water (1:1, 0.5 ml) was added to a suspension of *trans*-platin (24.7 mg, 0.082 mmol) in acetone/water (1:1, 0.5 ml) and sonicated for 24 h. The mixture was centrifuged and the supernatant was added to a solution of 1,4-bis(4-pyridyl)butadiyne (37.0 mg, 0.180 mmol) in acetone (0.5 ml), maintaining an excess of 1,4-bis(4-pyridyl)butadiyne. The mixture was sonicated for 24 h and then centrifuged to yield *trans*-diamminebis[1,4-bis(4-pyridyl)butadiyne]platinum(II) dinitrate (64.8 mg, 85%) as a light brown solid. $^1$H n.m.r. {500 MHz, DMSO}: δ 8.82, AA'm, 4H, H2, H6; 8.72, AA'm, 4H, H2', H6'; 7.99, BB'm, 4H, H3, H5; 7.65, BB'm, 4H, H3', H5'; 4.74, b s, 6H, $NH_3$. ESMS (CV=20 V): *m*/*z* 318.7 ($M^{2+}$, 80%).

**Bis-intercalators with extended linkers based on *trans*-platin. *trans*-Diamminebis(4,4'-dipyridyl)platinum(II) bis[(2,2':6',2''-terpyridine)platinum(II)] hexanitrate ($A_{20}$)**

A solution of silver nitrate (37.0 mg, 0.218 mmol) in acetone/water (4:1, 0.5 ml) was added dropwise to a suspension of diiodo-1,5-cyclooctadieneplatinum(II) (55.0 mg, 0.100 mmol) in acetone/water (4:1, 0.5 ml). The mixture was vortexed and sonicated for a few minutes and then centrifuged. The silver iodide precipitate was discarded. The supernatant was added to a suspension of 2,2':6',2''-terpyridine (18.7 mg, 0.081 mmol) in acetonitrile (0.25 ml). The mixture was vortexed and sonicated for a few minutes and then centrifuged. The supernatant was removed and discarded. The pellet was washed with acetonitrile/ether (1:3, 2x1.5 ml) and then dissolved in water (0.75 ml). A suspension of trans-diamminebis(4,4'-dipyridyl)platinum(II) nitrate (23.0 mg, 0.035 mmol) in methanol (0.75 ml) was added. The mixture was vortexed and sonicated for 5 days at room temperature. The mixture was centrifuged. The supernatant was added dropwise to ether/acetone (1:1, 20 ml) to precipitate the complex. Centrifugation yielded a purple solid (30.1 mg), which dissolved in water to give a yellow solution. Analysis of the $^1$H n.m.r. spectrum indicated the solid to be a complex mixture. Re-precipitation (5 times) from ether/acetone (1:1, 20 ml) gave the desired complex (8.3 mg) as a pale yellow solid. $^1$H n.m.r. {500 MHz, $D_2O$}: δ 9.38, AA'm, 4H, H2''', H6'''; 9.15, AA'm, 4H, H2'''', H6''''; 8.54, t, *J* 8.2 Hz, 2H, H4'; 8.46-8.41, series of multiplets, 12H, H3', H5', H3, H3'', H4, H4''; 8.35, BB'm, H3''', H5'''; 8.20, BB'm, 4H, H3'''', H5''''; 7.86, d, *J* 5.6 Hz, 4H, H6, H6''; 7.72, m, 4H, H5, H5''. ESMS (CV=10 V): *m*/*z* 242.5 {[M-Pt(terpy)]$^{4+}$, 9%}, 279.4 [$(M-H)^{5+}$, 100%], 292.2 {[(terpy)Pt(bip)]$^{2+}$, 35%}, 322.9 {[M-Pt(terpy)-H]$^{3+}$, 20%}.

***trans*-Diamminebis(4,4'-dipyridyl)platinum(II) bis[(4'-chloro-2,2':6',2''-terpyridine)platinum(II)] hexanitrate ($I_{20}$)**

A solution of silver nitrate (37.0 mg, 0.218 mmol) in acetone/water (4:1, 0.5 ml) was added dropwise to a suspension of diiodo-1,5-cyclooctadieneplatinum(II) (55.0 mg, 0.100 mmol) in acetone/water (4:1, 0.5 ml). The mixture was vortexed and sonicated for a few minutes and then centrifuged. The silver iodide precipitate was discarded. The supernatant was added to a suspension of 4'-chloro-2,2':6',2''-terpyridine (21.4 mg, 0.080 mmol) in acetonitrile (0.25 ml). The mixture was vortexed and sonicated for a few minutes and then centrifuged. The supernatant was removed and discarded. The pellet was washed with acetonitrile/ether (1:3, 2x1.5 ml) and then dissolved in water (0.75 ml). A suspension of *trans*-diamminebis(4,4'-dipyridyl)platinum(II) nitrate (23.0 mg, 0.035 mmol) in methanol (0.75 ml) was added. The mixture was vortexed and sonicated for 5 days at room temperature. The mixture was centrifuged. The supernatant was added dropwise to ether/acetone (1:1, 20 ml) to precipitate the complex. Centrifugation yielded a mauve solid (28.4 mg), which dissolved in water to give a yellow solution. Analysis of the $^1$H n.m.r. spectrum indicated the solid to be a complex mixture. Re-precipitation (4 times) from ether/acetone (1:1, 20 ml) gave the desired complex (12.1 mg) as a pale yellow solid. $^1$H n.m.r. {500 MHz, $D_2O$}: δ 9.37, AA'm, 4H, H2''', H6'''; 9.15, AA'm, 4H, H2'''', H6''''; 8.60, s, 4H, H3', H5'; 8.47-8.41, series of multiplets, 8H, H3, H3'', H4, H4''; 8.36, BB'm, H3''', H5'''; 8.20, BB'm, 4H, H3'''', H5''''; 7.89, d, *J* 5.7 Hz, 4H, H6, H6''; 7.76, m, 4H, H5, H5''. ESMS (CV=5 V): *m*/*z* 270.4 {[$Pt(bip)_2(NH_3)_2$]$^{2+}$, 28%},

293.2 [$(M-H)^{5+}$, 100%], 309.3 {[$(Cl\text{-}terpy)Pt(bip)]^{2+}$, 22%}, 334.2 {[$M\text{-}Pt(Cl\text{-}terpy)\text{-}H]^{3+}$, 48%}.

**References**

[1] Jennette, K., Lippard, S.J., Vassiliades, G. and Bauer, W. (1974) *Proc. Natl. Acad.Sci.USA*, **71,** 3839-3843.
[2] Bond, P.J., Langridge,R., Jennette,K.W. and Lippard,S.J.(1975) *Proc. Natl. Acad. Sci. USA,* **72**, 4825-29.
[3] Howe-Grant, M., Wu,K.C., Bauer,W.R and Lippard,S.J. (1976) *Biochemistry,* **15,** 4339-4346.
[4] Peyratout, C.S., Aldridge, T.K., Crites, D.K. and McMillin, D.R. (1995) *Inorg. Chem.*, **34**, 4484-89.
[5] McCoubrey, A., Latham, H.C., Cook, P.R., Rodger, A. and Lowe,G. *FEBS Letts.* 1996, **380,** 73-78
[6] Quigley, G.J., Wang, A.H.-J., Ughetto, G., van der Marel, G., van Boom, J.H. and Rich, A. *Proc. Natl. Acad. Sci. USA,* 1980, **77,** 7204.
[7] Lowe, G. and Vilaivan, T. *J.C.S. Perkin Trans. 1*, 1996, 1499-1503.
[8] Comess, K.M. and Lippard, S.J. in Molecular Aspects of Anticancer Drug-DNA Interactions Volume 1, ed. by Neidle, S. and Waring, M., The MacMillan Press, 1993, pp. 134-168.
[9] Hollis, L.S. Sundquist, W.I, Burstyn, J.N., Heiger-Bernays, W.J., Bellon, S.F., Ahmed, S.F., Amundsen, A.R., Stern, E.W. and Lippard, S.J., *Cancer Res.* 1991, **51**, 1866; Hollis, L.S., Amundsen, A.R. and Stern, E.W., *J. Med. Chem.,* 1989, **32,** 128.
[10] Lowe, G., McCloskey, J.A., Ni, J. and Vilaivan, T. *Bioorganic & Med. Chem.,* 1996, **4**,1007-1013.
[11] Matsumura, Y. and Maeda, H., *Cancer. Res.,* 1986, **6,** 6387-92; Maeda, H. and Matsumura, Y. *CRC Crit. Rev. Ther. Drug Carrier* Sys., 1989, **6**, 193-210
[12] Webb, A., Cunningham, D., Cotter, F., Clarke, P.A., DiStefano, F., Ross, P., Corbo, M. and Dziewanowska, Z., *The Lancet,* 1997, **349**, 1137-1141.
[13] ICON Collaborators, *The Lancet,* 1998, **352**, 1571-1576.
[14] Della Ciana, L., Haim, A., *J. Heterocyclic Chem.,* 1984, **21,** 607.

## Table 1

**The 96 hour $IC_{50}$ values (in μM) for the *in vitro* growth inhibition of human ovarian cell lines.**

Two of the cell lines are resistant to cisplatin and one to doxorubicin.
RF is the resistance factor: $IC_{50}$ resistance line/$IC_{50}$ parent line.

| Compound | CH1 | CH1cis$^R$ | RF | CH1dox$^R$ | RF | A2780 | A2780cis$^R$ | RF | SKOV3 |
|---|---|---|---|---|---|---|---|---|---|
| cisplatin | 0.4 | 1.2 | 3.0 | | | 0.53 | 8.8 | 16.6 | 2.25 |
| $A_{14}(4BF_4)$ | 1.35 | 0.63 | 0.46 | 5.1 | 3.8 | 1.6 | 2.4 | 1.5 | 1.3 |
| $A_{14}(4NO_3)$ | 2.05 | 2.15 | 1 | 0.71 | 0.3 | >25 | 11 | --- | 3.5 |
| $I_{14}(4BF_4)$ | 15.0 | 17.0 | 1.1 | 7.8 | 0.5 | --- | 19 | --- | 15.0 |
| $Q_{14}(4BF_4)$ | 2.0 | 2.8 | 1.4 | 1.9 | 0.9 | --- | 2.3 | --- | 4.7 |
| $A_{15}(4NO_3)$ | 0.73 | 0.73 | 1 | 0.44 | 0.6 | 2 | 1.8 | 0.9 | 1.7 |
| $I_{15}(4NO_3)$ | 1.3 | 1.75 | 1.3 | --- | --- | 0.62 | 0.68 | 1.1 | 1.4 |
| $I_{16}(4NO_3)$ | 2.15 | 2.15 | 1 | --- | --- | 4.75 | 6.6 | 1.4 | 1.8 |
| $I_{17}(4NO_3)$ | 2.5 | 3.1 | 1.2 | --- | --- | 8.6 | 12.5 | 1.4 | 2.2 |
| $A_{18}(4NO_3)$ | 1.55 | 2.05 | 1.3 | 0.56 | 0.4 | >25 | 9.6 | --- | 3.1 |
| $I_{18}(4NO_3)$ | 0.59 | 0.87 | 1.5 | 0.69 | 1.1 | >25 | 18.5 | --- | 1.3 |
| $A_{20}(6NO_3)$ | 1.4 | 1.8 | 1.3 | 0.46 | 0.3 | 2.4 | 1.4 | 0.6 | 5.1 |
| $I_{20}(6NO_3)$ | 0.55 | 0.81 | 1.5 | 0.42 | 0.8 | 13.5 | 20.5 | 1.5 | 1.7 |
| carboplatin | 6.2 | 14.0 | 2.3 | --- | --- | 35 | >100 | --- | >100 |

## Claims

1. A 2, 2':6', 2"-terpyridine Pt(II) complex of the structure

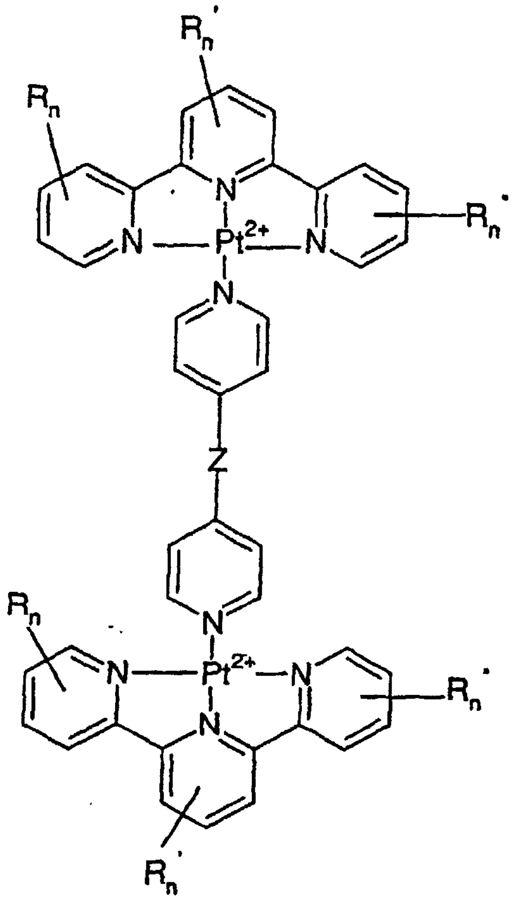

where R, R' and R" are the same or different and each is H, alkyl, aryl, aralkyl, alkaryl, acyl, halogen, haloalkyl, haloaryl, hydroxyalkyl, hydroxyaryl, aminoalkyl, aminoaryl, primary secondary or tertiary amino hydrazino, alkylhydrazino, alkoxy, aralkoxy, nitrite, ester, amido, nitro, azido or aziridino,
n is 1, 2 or 3,
Z is a linker composed of 0 to 6 moieties selected from -CH=CH-
-C≡C- , o-, m- or p- $-C_6H_3R'''-$ , and Q,
Q is $-C_5H_3R'''N-Pt^{2+}(NH_3)_2-NC_5H_3R'''-$,
R''' is defined as R, R' and R",
and a counterion to make the complex electrically neutral,
provided that not more than one Q is present,
and provided that Z is not -CH=CH-

2. The complex of claim 1, wherein the counterions are selected from $BF_4^-$, nitrate, sulphate, carbonate, phosphate, pyrophosphate or other phosphorus oxyanion or ester thereof, sulphonates and carboxylates.

3. The complex of claim1 or claim 2, wherein R is H, R" is H, and R' is 4'-H or 4'-C1 and n is 1.

4. The complex of any one of claims 1 to 3, wherein Z is either 1 to 6 moieties selected from -C≡C- or Q, or a direct bond.

5. The complex of any one of claims 1 to 3, wherein Z is $-C \equiv C- (C_6H_4)_m -C \equiv C-$, where m is 0, 1 or 2.

6. The complex of any one of claims 1 to 3, wherein Z is a direct bond or Q and R''' is H.

7. The complex of any one of claims 1 to 3 wherein Z is a direct bond.

8. The complex of any one of claims 1 to 7, which the tetranitrate of 1,4-bis(4-pyridyl)butadiyne bis[2,2':6',2"-terpyridine) platinum (II)] ($A_{15}$), 1,4-bis(4-pyridyl butadiynebis[4'chloro-2,2':6'2"-terpyridine) platinum II] ($I_{15}$), 4,4"-dipyridyl -1'4'-diethynyl benzene -bis [(4'-chloro-2,2':6',2"-terpyridine)platinum(II)]($I_{16}$), [(4,4'-diethynyl -4",4"'-dipyridyl-biphenyl-bis[4'-chloro-2, 2':6', 2"-terphyridine) platinum (II) ($I_{17}$), 4,4'-dipyridine bis [(2,2':6', 2"-terpyridine) platinum II] ($A_{18}$), 4, 4'-dipyridine bis[(4'-chloro-2,2':6',2"-terpyridine) platinum (II) ($I_{18}$), trans-diammine bis (4,4'-dipyridyl) platinum (II) bis [(2, 2':6',2"-terpyidine platinum (II) ($A_{20}$) or trans-diammine bis(4,4'-dipyridyl platiaum (II) bis [(4'-chloro-2,2':6',2"-terpyridine platinum (II) ($I_{20}$).

9. A method of preparing an anti-tumour agent, which method comprises bringing the complex of any one of claims 1 to 8 into a form suitable for administration.

10. A conjugate of the 2,2':6',2"-terpyridine Pt(II) complex of any one of claims 1 to 8 with an anionic polymer or dendrimer.

11. A preparation suitable for use in the treatment of cancer which comprises a complex as claimed in any one of claims 1 to 8 or a conjugate as claimed in claim 10.

12. A preparation according to claim 11 which comprises an agent which down-regulates expression of Bcl-2 gene.

13. A complex as defined in any one of claims 1 to 8 or a conjugate as defined in claim 10 for use in the treatment of cancer.

## Patentansprüche

1. 2,2':6',2"-Terpyridin-Pt(II)-Komplex der folgenden Struktur

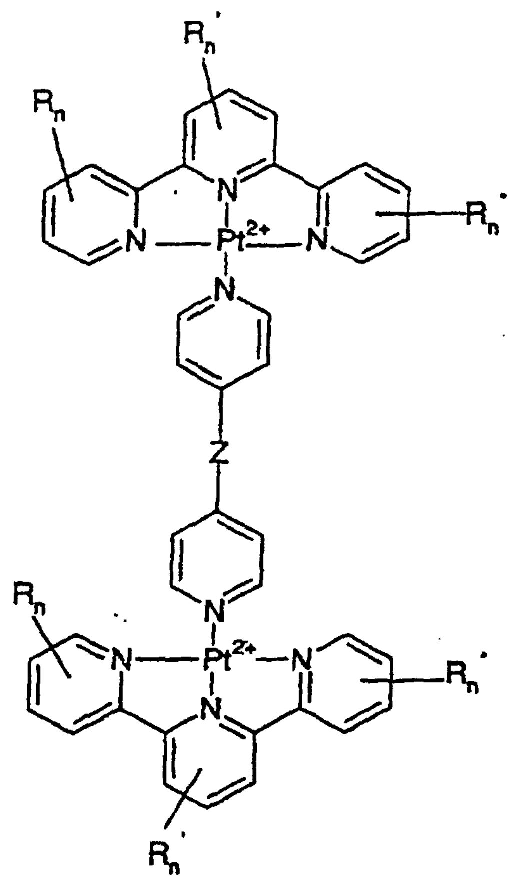

worin R, R' und R" gleich oder verschieden sind und jedes H, Alkyl, Aryl, Aralkyl, Alkaryl, Acyl, Halogen, Haloalkyl, Haloaryl, Hydroxyalkyl, Hydroxyaryl, Aminoalkyl, Aminoaryl, primäres, sekundäres oder tertiäres Aminohydrazino, Alkylhydrazino, Alkoxy, Aralkoxy, Nitril, Ester, Amido, Nitro, Azido oder Aziridino ist,
n 1, 2, oder 3 ist,
Z ein Linker ist, der sich aus 0 bis 6 Komponenten zusammensetzt, gewählt aus -CH=CH-, -C≡C-, o-, m- oder p-, $-C_6H_3R'''$- und Q,
Q ist $-C_5H_3R'''N-Pt^{2+}(NH_3)_2-NC_5H_3R'''-$,
R''' als R, R' und R" definiert ist,
und ein Gegenion, um den Komplex elektrisch neutral zu machen, vorausgesetzt, dass nicht mehr als ein Q vorhanden ist, und vorausgesetzt, dass Z nicht -CH=CH- ist.

2. Komplex nach Anspruch 1, wobei die Gegenionen gewählt sind aus $BF_4^-$, Nitrat, Sulfat, Carbonat, Phosphat, Pyrophosphat oder einem anderen Phosphoroxyanion oder Ester davon, Sulfonaten und Carboxylaten.

3. Komplex nach Anspruch 1 oder Anspruch 2, worin R H ist, R" H ist und R' 4'-H oder 4'-Cl ist und n 1 ist.

4. Komplex nach einem der Ansprüche 1 bis 3, worin Z entweder 1 bis 6 Komponenten, gewählt aus -C≡C- oder Q, oder eine direkte Bindung ist.

5. Komplex nach einem der Ansprüche 1 bis 3, worin Z $-C{\equiv}C-(C_6H_4)_m-C{\equiv}C-$ ist, worin m 0, 1 oder 2 ist.

6. Komplex nach einem der Ansprüche 1 bis 3, worin Z eine direkte Bindung ist oder Q und R''' H ist.

7. Komplex nach einem der Ansprüche 1 bis 3, worin Z eine direkte Bindung ist.

8. Komplex nach einem der Ansprüche 1 bis 7, welcher das Tetranitrat von 1,4-Bis(4-pyridyl)butadiin-bis[2,2':6',2"-terpyridin)platin(II)] ($A_{15}$), 1,4-Bis(4-pyridyl)butadiin-bis[4'-chloro-2,2':6',2"-terpyridin)platin(II)] ($I_{15}$), 4,4"-Dipyridyl-1',4'-diethynylbenzol-bis[(4'-chloro-2,2':6',2"-terpyridin)platin(II)] ($C_{16}$), 4,4'-Diethynyl-4",4'''-dipyridylbiphenyl-bis[4'-chloro-2,2':6',2"-terpyridin)platin(II) ($I_{17}$), 4,4'-Dipyridin-bis[(2,2':6',2"-terpyridin)platin(II)] ($A_{18}$), 4,4'-Dipyridin-bis[(4'-chloro-2,2':6',2"-terpyridin)platin(II) ($I_{18}$), trans-Diammin-bis(4,4'-dipyridyl)platin(II)-bis[(2,2':6',2"-terpyridin)platin(II) ($A_{20}$) oder trans-Diammin-bis(4,4'dipyridyl)platin(II)-bis[(4'-chloro-2,2':6',2"-terpyridin)platin(II) ($I_{20}$).

9. Verfahren zur Herstellung eines Antitumormittels, welches Verfahren das Bringen des Komplexes nach einem der Ansprüche 1 bis 8 in eine zur Verabreichung geeignete Form umfasst.

10. Konjugat des 2,2':6',2"-Terpyridin-Pt(II)-Komplexes nach einem der Ansprüche 1 bis 8 mit einem anionischen Polymer oder Dendrimer.

11. Präparat, das sich zur Anwendung in der Behandlung von Krebs eignet, welches einen Komplex nach einem der Ansprüche 1 bis 8 oder ein Konjugat nach Anspruch 10 umfasst.

12. Präparat nach Anspruch 11, welches ein Mittel umfasst, das die Expression des Bcl-2-Gens herabreguliert.

13. Komplex, wie in einem der Ansprüche 1 bis 8 definiert, oder ein Konjugat, wie in Anspruch 10 definiert, zur Anwendung in der Behandlung von Krebs.

**Revendications**

1. Complexe 2,2':6',2"-terpyridine Pt(II) ayant la structure :

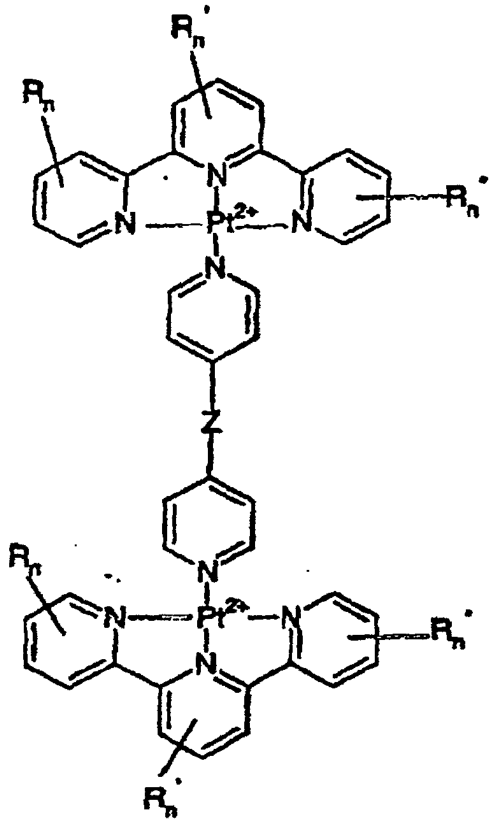

dans laquelle R, R' et R" sont identiques ou différents et chacun représente H, un alkyle, un aryle, un aralkyle, un alcaryle, un acyle, un atome d'halogène, un halogénoalkyle, un halogénoaryle, un hydroxyalkyle, un hydroxyaryle, un aminoalkyle, un aminoaryle, une amine primaire, secondaire ou tertiaire, un hydrazino, un alkylhydrazino, un alcoxy, un aralcoxy, un nitrile, un ester, un amido, un nitro, un azido ou un aziridino,

n vaut 1, 2 ou 3,

Z représente un groupe liant composé de 0 à 6 fragments choisis parmi -CH=CH-, -C≡C-, o-, m- ou p- $-C_6H_3R'''-$ et Q,

Q représente $-C_5H_3R'''N-Pt^{2+}(NH_3)_2-NC_5H_3R'''-$ ,

R"' est défini comme R, R' et R",

et un contre-ion pour rendre le complexe électriquement neutre, à condition que pas plus d'un Q soit présent, et à condition que Z ne soit pas -CH=CH-.

2. Complexe selon la revendication 1, dans lequel les contre-ions sont choisis parmi $BF_4^-$, le nitrate, le sulfate, le carbonate, le phosphate, le pyrophosphate ou un autre oxyanion de phosphore ou un ester de celui-ci, des sulfonates et des carboxylates.

3. Complexe selon la revendication 1 ou 2, dans lequel R représente H, R" représente H et R' représente 4'-H ou 4'-Cl et n vaut 1.

4. Complexe selon l'une quelconque des revendications 1 à 3, dans lequel Z représente soit 1 à 6 fragments choisis parmi -C≡C- ou Q, soit une liaison directe.

5. Complexe selon l'une quelconque des revendications 1 à 3, dans lequel Z représente -C≡C- $(C_6H_4)_m$-C≡C-, où m vaut 0, 1 ou 2.

6. Complexe selon l'une quelconque des revendications 1 à 3, dans lequel Z représente une liaison directe ou Q et R"' représente H.

7. Complexe selon l'une quelconque des revendications 1 à 3, dans lequel Z représente une liaison directe.

**8.** Complexe selon l'une quelconque des revendications 1 à 7, dont le tétranitrate de 1,4-bis(4-pyridyl)butadiyne bis [2,2':6',2"-terpyridine)-platine (II)]($A_{15}$), le 1,4-bis(4-pyridyl)butadiyne bis[4'-chloro-2,2':6',2"-terpyridine)platine (II)] ($I_{15}$), le 4,4"-dipyridyl-1',4'-diéthynyl benzène-bis[(4'-chloro-2,2':6',2"-terpyridine)platine (II)]($I_{16}$), 4,4'-diéthynyl-4", 4'''-dipyridyl-biphényl-bis[4'-chloro-2,2':6',2"-terpyridine)platine (II)]($I_{17}$), 4,4'-dipyridine-bis[(2,2':6',2"-terpyridine) platine (II)]($A_{18}$), 4,4'-dipyridine-bis[(4'-chloro-2,2':6',2"-terpyridine)platine (II)] ($I_{18}$), trans-diamine bis(4,4'-dipyridyl) platine (II) bis[(2,2':6',2"-terpyridine)platine (II)] ($A_{20}$) ou trans-diamine bis(4,4'-dipyridyl) platine (II) bis[(4'-chloro-2,2':6',2"-terpyridine)platine (II)] ($I_{20}$).

**9.** Procédé de préparation d'un agent anti-tumoral lequel procédé comprend le fait de donner au complexe selon l'une quelconque des revendications 1 à 8 une forme appropriée pour une administration.

**10.** Conjugué du complexe 2,2':6',2"-terpyridine Pt(II) selon l'une quelconque des revendications 1 à 8 avec un dendrimère ou un polymère anionique.

**11.** Préparation appropriée pour une utilisation destinée au traitement d'un cancer qui comprend un complexe selon l'une quelconque des revendications 1 à 8 ou un conjugué selon la revendication 10.

**12.** Préparation selon la revendication 11 qui comprend un agent qui régule à la baisse l'expression du gène Bc1-2.

**13.** Complexe selon l'une quelconque des revendications 1 à 8 ou conjugué selon la revendication 10 pour une utilisation destinée au traitement d'un cancer.

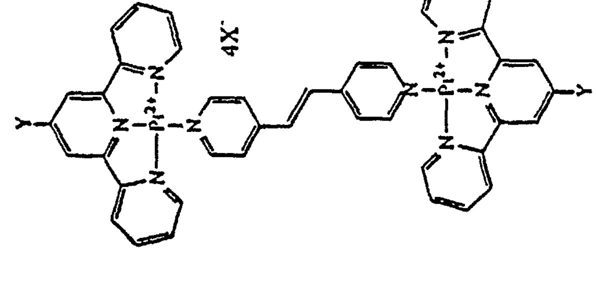

$4X^-$

$A_{14}$, Y=H, X=$NO_3$ or $BF_4$
$I_{14}$, Y=Cl, X=$NO_3$ or $BF_4$

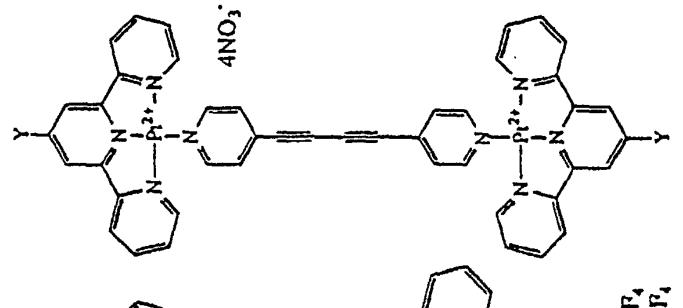

$4NO_3^-$

$A_{15}$, Y=H
$I_{15}$, Y=Cl

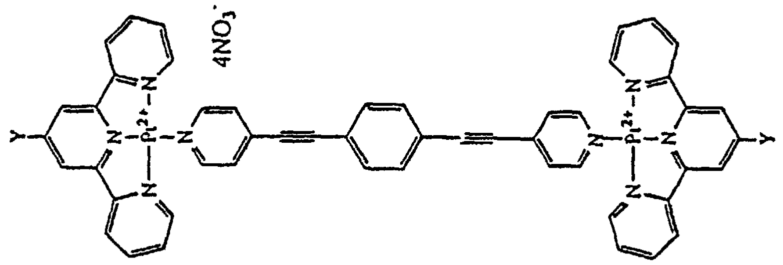

$4NO_3^-$

$I_{16}$ Y=Cl

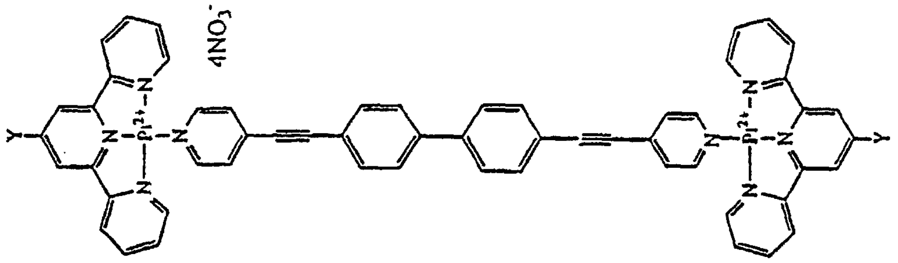

$4NO_3^-$

$I_{17}$, Y=Cl

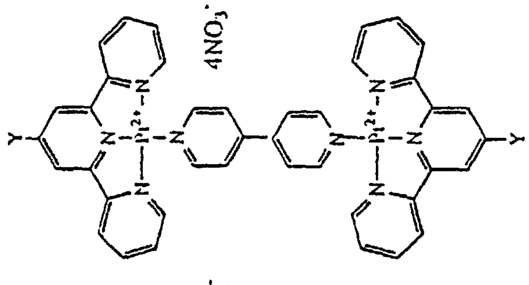

$4NO_3^-$

$A_{18}$ Y=H
$I_{18}$ Y=Cl

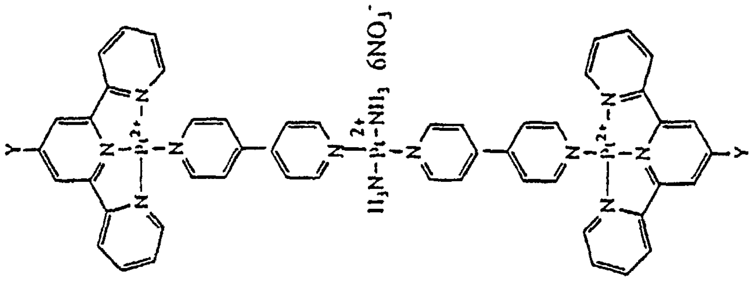

$6NO_3^-$

$A_{20}$ Y=H
$I_{20}$ Y=Cl

Figure 1